(19) Europäisches Patentamt
European Patent Office
Office européen des brevets

(11) **EP 2 632 341 B1**

(12) **EUROPEAN PATENT SPECIFICATION**

(45) Date of publication and mention of the grant of the patent:
**20.10.2021 Bulletin 2021/42**

(51) Int Cl.:
*A61B 8/14* *(2006.01)*          *A61B 8/00* *(2006.01)*
*A61B 8/08* *(2006.01)*          *A61B 8/12* *(2006.01)*

(21) Application number: **11853390.0**

(22) Date of filing: **13.09.2011**

(86) International application number:
**PCT/US2011/051446**

(87) International publication number:
**WO 2012/091764 (05.07.2012 Gazette 2012/27)**

(54) **IDENTIFICATION OF OBJECTS IN ULTRASOUND**

ULTRASCHALLIDENTIFIZIERUNG VON OBJEKTEN

IDENTIFICATION D'OBJETS DANS UNE IMAGE ULTRASONORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2010 US 978935**

(43) Date of publication of application:
**04.09.2013 Bulletin 2013/36**

(73) Proprietor: **St. Jude Medical,
Atrial Fibrillation Division, Inc.
St. Paul, Minnesota 55117-9913 (US)**

(72) Inventors:
• **HILL, Anthony**
  **Minneapolis, Minnesota 55407 (US)**
• **DENO, D. Curtis**
  **Andover, Minnesota 55304 (US)**
• **AIKEN, Robert D.**
  **Stillwater, Minnesota 55082 (US)**
• **ZHONG, Hua**
  **Pittsburgh, Pennsylvania 15217 (US)**
• **KUDAS, Mark**
  **Astoria, New York 11102 (US)**

(74) Representative: **Kramer Barske Schmidtchen
Patentanwälte PartG mbB
European Patent Attorneys
Landsberger Strasse 300
80687 München (DE)**

(56) References cited:
EP-A1- 1 504 713          EP-A1- 2 264 670
US-A- 5 182 728          US-A1- 2005 203 394
US-A1- 2006 122 514          US-B1- 6 556 695

• None

**Description**

BACKGROUND OF THE INVENTION

a. Field Of The Invention

The present disclosure relates to catheter devices and systems, including devices and methods for automatically identifying anatomic objects in an ultrasound image.

b. Background Art

Electrophysiology (EP) catheters are used in connection with an ever-increasing number of procedures. Such catheters have been used, for example, for diagnostic, therapeutic, mapping, and ablative procedures. Catheters are commonly manipulated through a patient's vasculature to an intended site, for example a site within the patient's heart, and may carry one or more ultrasound transducers, position sensors, or electrodes for use in sensing, mapping, ablation, or diagnosis procedures.

US 2005/203394 A1 relates to a system and method for navigating an ultrasound catheter to image beating heart, in particular by coordination of catheter position data with ultrasound imaging data imaging a heart via ultrasound.

EP 1 504 713 A1 relates to image guided surgery and more particular to systems and methods for using one or more medical images to assist in navigating an instrument through internal body structures.

US 2006/122514 A1 relates to medical imaging systems for localizing an ultrasound imaging catheter.

BRIEF SUMMARY OF THE INVENTION

A method for identifying objects in an ultrasound image according to claim 1 is disclosed.

In an embodiment, the ultrasound information is registered to the 3-D cardiac model by sensing a signal from a position sensor associated with the catheter indicative of a position and orientation of the ultrasound information, and then using the signal to locate the ultrasound information within the three-dimensional cardiac model. In an embodiment, the position sensor may be either an electrode configured to respond to an electric field, or a coil that is responsive to a magnetic field.

Distinct objects are displayed within a visual representation of the ultrasound information by extracting two-dimensional boundary information from the three-dimensional cardiac model that is coincident between the model and the registered ultrasound information, and then displaying a two-dimensional augmented echo image that includes a visual representation of ultrasound information along with an overlay of the extracted, coincident boundary information. The boundary information is visualized as boundary lines, and may be identified using the same identifiers provided for the features within the 3-D model. Similarly, in an embodiment, electrophysiological markers or markers denoting physical points of interest, ablation lesions, catheters, electrodes, or magnetically responsive coils, may be extracted from the three-dimensional cardiac model and overlaid on the visual representation of the ultrasound information.

BRIEF DESCRIPTION OF THE DRAWINGS

FIG. 1 is a general representation of a cardiac anatomy together with a catheter.

FIG. 2a is a general representation of a volumetric cardiac model including a representation of a phased array catheter.

FIG. 2b is a general representation of an augmented echo image including a phased array ultrasound image and overlaid boundary information.

FIG. 3a is a general representation of a volumetric cardiac model including a representation of a radially scanning catheter.

FIG. 3b is a general representation of an augmented echo image including a radially scanning ultrasound image and overlaid boundary information.

FIG. 4a is a general representation of a volumetric cardiac model including a representation of a phased array catheter, electrodes, and a point of interest

FIG. 4b is a general representation of an augmented echo image including a phased array ultrasound image and overlaid boundary information, electrodes, and a point of interest.

FIG. 5 is a flow chart illustrating a method of identifying features via ultrasound.

FIG. 6 is a schematic diagram illustrating a system for identifying objects via ultrasound.

FIG. 7a is representation of a volumetric cardiac model including a representation of a phased array catheter.

FIG. 7b is a representation of an augmented echo image including a phased array ultrasound image and overlaid boundary information.

FIG. 8 is a display showing two views of a volumetric cardiac model, along with an augmented echo image including phased array ultrasound image and overlaid boundary information.

## DETAILED DESCRIPTION OF THE INVENTION

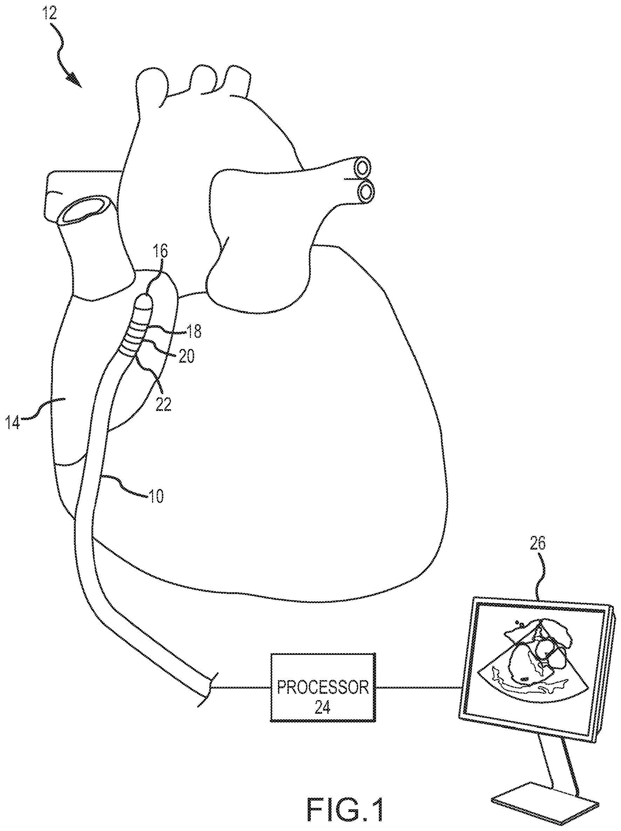

Referring to the drawings, wherein like reference numerals are used to identify like or identical components in the various views, Fig. 1 generally illustrates a catheter 10 positioned within a portion of a cardiac anatomy 12. As generally illustrated in Fig. 1, the catheter 10 may, for example, be positioned within the right atrium 14 of the cardiac anatomy 12. In an embodiment, the catheter 10 may be an intracardiac echo (ICE) catheter that may include one or more ultrasound transducers, such as ultrasound transducer 16. The catheter 10 may further include one or more position detectors 18, 20, 22 located toward its distal end, and configured to provide a signal indicative of both a position and orientation of a portion of the catheter 10.

In an embodiment, the position detectors 18, 20, 22, may be electrodes (e.g., ring-type or spot type or partially masked electrodes) configured to be responsive to an electric field transmitted within the body of the subject. Such electrodes may be used to sense an impedance at a particular location and transmit a representative signal to an external computer or processor. An example of an impedance-based position detection system is the EnSite NavX™ System, commercialized by St. Jude Medical, Inc. of St. Paul, MN, and described in U.S. Patent No. 7,263,397, entitled "Method And Apparatus For Catheter Navigation And Location And Mapping In The Heart".

In an embodiment, one or more of the position detectors 18, 20, 22 may be metallic coils located on or within the catheter 10, and may be configured to be responsive to a magnetic field transmitted through the body of the subject. Multiple coils may be oriented on different axes. Such coils may sense the strength of the field at a particular location and transmit a representative signal to an external computer or processor. An example of a magnetic-based position detection system is the Medical Positioning System (gMPS) for navigation developed by St. Jude Medical, Inc. through its MediGuide Inc. business unit of Haifa, Israel, and generally shown and described in US Patent No. 7,386,339 entitled "Medical Imaging and Navigation System".

The position detection system may also be a combination of one or more magnetic coils with one or more electrodes.

The ultrasound transducer 16 may be configured to project an ultrasound signal outward through the adjoining tissue and fluid, and may further receive the ultrasound echo from such tissue and fluid. In an embodiment, the ultrasound transducer 16 may be a unidirectional phased array ultrasound transducer. Such a transducer may be configured to project ultrasound energy from one side of the catheter in a two dimensional plane aligned with the longitudinal axis of the catheter. In another embodiment, the ultrasound transducer 16 may be a radially scanning ultrasound transducer that is configured to project ultrasound energy radially outward from the catheter and further configured to rotate about the circumference of the catheter through 360 degrees.

The system may additionally include a processor 24 and a display device 26. The processor, among other processing tasks, may be configured to receive position and/or orientation signals from the one or more position sensors associated with the distal end portion of the catheter (e.g. position sensors 18, 20, 22), may receive ultrasound information from the one or more ultrasound transducers (e.g. ultrasound transducer 16), may maintain a three-dimensional volumetric model of the cardiac anatomy, and may provide various displays to a display device 26.

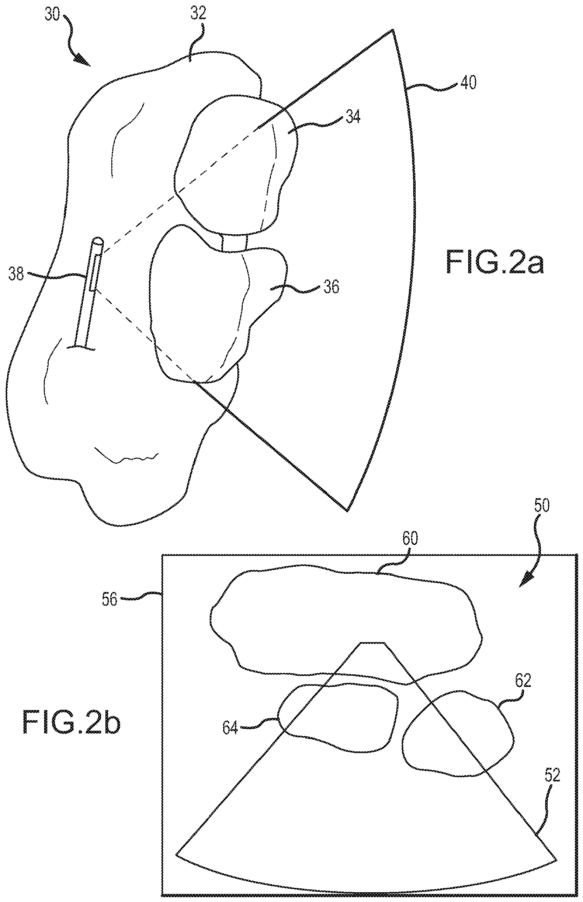

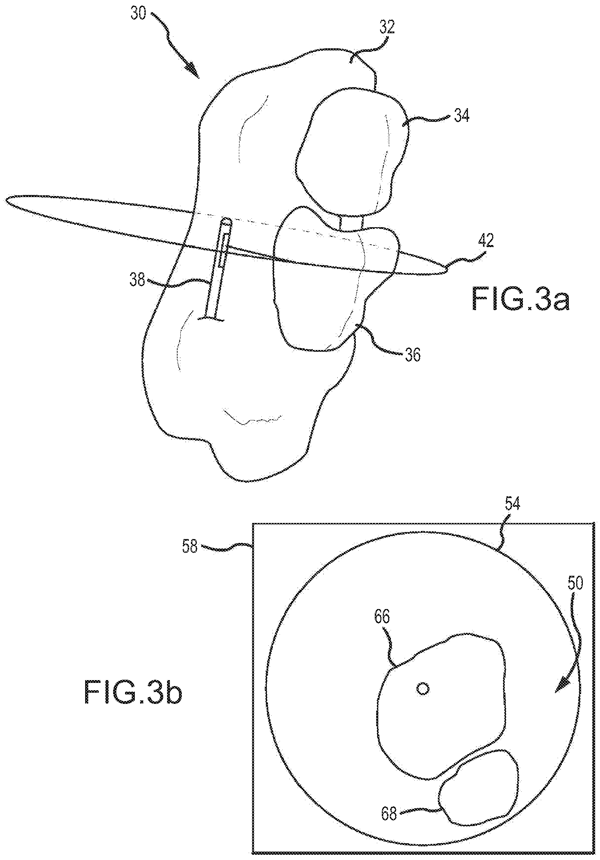

Figs. 2a and 3a both illustrate an exemplary three-dimensional cardiac model 30 that may reside in or be maintained by the processor 26. The cardiac model 30 illustrated in Figs. 2a and 3a, is simplified for the purpose of illustration. However, in practice, the cardiac model 30 may be more complex and/or representative of the actual cardiac anatomy 12. In an embodiment, the cardiac model 30 may represent the walls of an actual cardiac anatomy using a representative shell with a negligible thickness. In another embodiment, the walls may have a visible thickness representative of actual cardiac tissue. The cardiac model 30 may be readily displayable to a user through the use of a display device 26, such as a standard computer display.

In an embodiment, the three dimensional model 30 may be a segmented model that is constructed from two dimensional slice data obtained from computed tomography (CT) scans of a patient's anatomy 12. In another embodiment, a 3-D cardiac model 30 may be constructed from two-dimensional magnetic resonance imaging (MRI) slice data. The slice data may, for example, be automatically assembled into the segmented 3-D model using a computerized tool such as the EnSite Verismo™ Segmentation Tool, commercialized by St. Jude Medical, Inc. In yet another embodiment, the 3-D model may be generated from rotational angiography. In yet another embodiment, the 3-D model may be directly created by moving catheters inside the heart and monitoring the motion with a 3-D mapping system such as the EnSite™ NavX System. By examining the recorded motion, a 3-D shell may be fit to the outermost points.

The cardiac model 30 may include one or more identifiable objects that may be defined within the coordinate space of the model (e.g., a cartesian space). In an embodiment, the one or more objects may include structural and non-structural objects. Structural objects, such as volumetric regions of the model (e.g., structures 32, 34, 36), may represent specific portions of the subject's cardiac anatomy, such as the right atrium, left atrium, right ventricle, left ventricle, aorta, pulmonary vein, and/or pulmonary artery. Other non-structural objects that may incorporated within the cardiac model 30 include, for example, anatomical points/regions of interest (e.g., the pulmonary ostium or sinoatrial (SA) node), non-anatomic objects of interest (e.g., catheters or electrodes), and other labels or markers placed in the model by the physician (e.g., intended ablation sites, lesion markers, identifying labels).

Each identifiable object may likewise have one or more properties that define characteristics of a portion of the object or the object as a whole. In an embodiment, the properties may relate to the entire object, for example, a numeric object identifier that may be used to distinguish one object from another. Alternatively, the properties may be location specific and, for example, may simply define characteristics of a single point or portion of the object. These location specific properties may include, for example, activation time or electrogram amplitude of the cardiac tissue at a point on the volumetric structure, a measure of electrical coupling between a particular electrode and adjacent tissue (provided at the particular electrode), a measure of distance between a particular electrode and the cardiac anatomy, or a measure of the ablation energy or time at a particular point on the volumetric structure. In an embodiment where each point **(P)** within the cardiac model 30 is physically defined in three dimensions, the point may be represented, for example, by an array as shown in Equation 1, where ($x, y, z$) represent the location of the point in three dimensions, and ($C_1, C_2, \ldots, C_n$) represent the one or more properties at that location. Similar definitions may exist for entire objects and object-defined properties.

$$\mathbf{P} = [x,\, y,\, z,\, C_1,\, C_2,\, \ldots ,\, C_n] \qquad \text{Eq. 1}$$

Within the cardiac model 30, each object may be separately identified through the use of a distinct display color or textual label. In an embodiment, each identifier may uniquely relate to a numerical identifier property for that respective object. For example, and without limitation, within the display of cardiac model 30, structure 32 may be shaded a red color, structure 34 may be shaded a blue color, and structure 36 may be shaded a green color. Alternatively, structure 32 may be identified with the textual label "Right Atrium" or "RA," structure 34 may be identified with the textual label "Pulmonary Artery" or "PA," and structure 36 may be identified with the textual label "Aorta" or "Ao." Likewise, other identifiable objects may be identified with other unique colors and/or textual labels.

Instead of being identified by an object specific property, each object within the cardiac model 30 may be represented by a location specific property, such as a plurality of colors indicative of the magnitude or nature of a property at a plurality of points. For example, and without limitation, the color of every point on a volumetric structure may reflect the activation time of the cardiac tissue at each point (for example, as with an electrophysiological map of the cardiac tissue). In such an embodiment, a spectrum of colors may be used to represent a range of time values, where, for example, redder colors may reflect a greater activation time and bluer colors may reflect a lower activation time.

In the case where the cardiac model 30 is created using a separate imaging modality, such as CT or MRI, the model 30 may be registered to an actual cardiac anatomy 12 using a position sensing system with one or more position detectors (e.g., position detectors 18, 20, 22). If the model was created directly through the use of the positioning system, the registration of the model 30 to the actual cardiac anatomy 12 may not be necessary, or may be implicit. In an embodiment, registering a cardiac model may be performed by moving a catheter 10 through a series of known locations and recording the position at each location. The system may correlate the recorded positions to similar positions within an associated 3-D cardiac model 30, and adjust the rotation, offset, and/or scaling of the cardiac model 30 accordingly. In an embodiment, a physician may manipulate the catheter to known locations using personal experience with such procedures, or with the aid of real-time imaging, such as fluoroscopy. In an embodiment, the physician may use a dynamic registration tool, such as the EnSite Fusion™ Registration Tool, commercialized by St. Jude Medical, Inc., to accomplish the registration between a cardiac model 30, and an actual cardiac anatomy 12.

After the cardiac model 30 is registered to an actual cardiac anatomy 12 (either explicitly or implicitly), the system may incorporate a representation of a catheter 38 within the cardiac model 30. The catheter representation 38 may be positioned and oriented within the model 30 based on the position and orientation of the actual catheter 10. Furthermore, by understanding the physical relationship between one or more position sensors (e.g., position sensors 18, 20, 22) and the ultrasound transducer 16, the system may derive an exact position and orientation of the 2-D ultrasound spread. Knowledge of this positional relationship may then allow the system to locate a corresponding two-dimensional ultrasound image 40, 42 within the cardiac model 30, e.g., as shown in Figs. 2a and 3a. The ultrasound image 40, 42 displayed within the 3-D cardiac model 30 may be representative of the ultrasound information obtained by the transducer 16; and, for example, may, if desired, display tissues of greater acoustic reflectivity using increasingly bright monochrome colors.

Fig. 2a illustrates an embodiment in which the ultrasound transducer 16 is a phased array ultrasound transducer.

As generally illustrated, the ultrasound image 40 projects out directionally from a portion of the catheter representation 38. Fig. 3 a illustrates an embodiment where the ultrasound transducer 16 is a radially scanning ultrasound transducer. As generally illustrated in Fig. 3a, the ultrasound image 42 projects out radially from the catheter 38 in a manner that may be generally orthogonal to the axis of the catheter 38.

Once a two-dimensional ultrasound image 40, 42 is located within the cardiac model 30, the image may be used to generally define a model slice plane. As used herein, the model slice plane may be a two-dimensional plane, located within a three-dimensional model-space, that contains the two-dimensional ultrasound image 40, 42, and further intersects the representation of the catheter 38 and a portion of the cardiac model 30.

The system may use the model slice plane for the purpose of extracting model information that lies within the plane of the ultrasound image. In an embodiment, the intersection of the model slice plane and cardiac model may create a set of boundary lines that represent the walls of a cardiac anatomy within that plane. As shown generally in Figs. 2b and 3b, the boundary information 50 may then be overlaid on an independent visualization of the ultrasound information 52, 54 to create an augmented echo image 56, 58.

In an embodiment employing a phased array ultrasound transducer, such as generally illustrated in Fig. 2b, the augmented echo image 56 may contain a first boundary marker 60 that corresponds to structure 32 depicted in Fig. 2a. Likewise a second boundary marker 62 may correspond to structure 34, and a third boundary marker 64 may correspond to structure 36.

In an embodiment employing a radially scanning ultrasound transducer, as generally illustrated in Fig, 3b, an augmented echo image 58 may contain a first boundary marker 66 that may correspond to structure 32 depicted in Fig. 3a, and a second boundary marker 68 may correspond to structure 36. Since the ultrasound plane (and hence the model slice plane) does not intersect structure 34 (as illustrated in Fig. 3a), it is not represented within the augmented echo image 58.

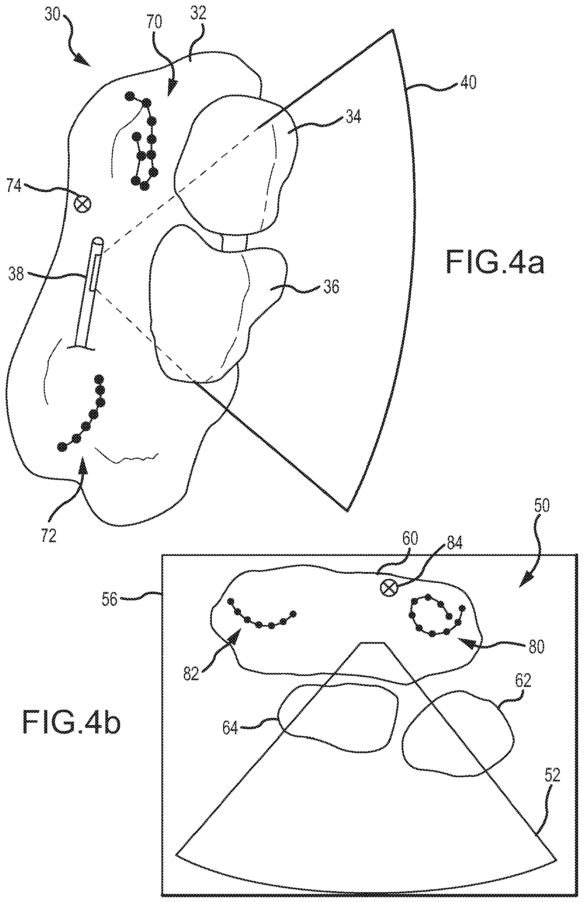

In addition to identifying structural objects within the plane of the ultrasound image, the system may also be configured to extract other non-structural objects located within a given tolerance of the model slice plane, as generally shown in Figs. 4a and 4b. As previously described, such other objects may include markers identifying physical points or areas of interest within the cardiac anatomy (e.g., the SA node and/or pulmonary ostium), electrophysiological markers identifying items specific to a procedure (e.g., ablation lesion markers and/or markers denoting tissue with a specific electrophysiological property), or a representation of a catheter, electrode, or magnetically responsive coil.

Fig. 4a illustrates a generalized 3-D cardiac model 30 that includes an identification of other non-structural objects (i.e., catheter electrodes 70, 72, and marker 74) within the cardiac model. As described above, a two-dimensional ultrasound image 40 may be located within the cardiac model 30, and may be used to generally define a model slice plane. The system may use the model slice plane for the purpose of identifying anatomic structure within the plane of the ultrasound image. Additionally, the system may examine the model and extract other, non-structural objects that exist within a given tolerance of the model slice plane. Such features not lying within the model slice plane may be orthogonally projected to the plane for the purpose of constructing the overlay and augmented echo image 56. As shown in Fig. 4b, catheter electrodes 70 within the model may be displayed as electrodes 80 within the augmented echo image. Likewise, electrodes 72 may be displayed as electrodes 82, and marker 74 may be displayed as marker 84.

To aid a physician or other medical professional in readily identifying objects represented in the augmented echo image (e.g. augmented echo image 56, 58), the system may, upon instruction or automatically, identify each object in the augmented echo image (e.g., structures 60, 62, 64 in Fig. 2b) using the same or a similar unique identifier provided for that object in the cardiac model 30. For example, with reference to Figs. 2a and 2b, if structure 32 was shaded a red color, the corresponding boundary marker 60 within the augmented echo image 56 may be displayed using the same (or similar) red color. Likewise if structure 34 was shaded a blue color, the corresponding boundary marker 62 may be displayed using a similar blue color; and if structure 36 were green, boundary marker 64 may be displayed using a similar green color. In another embodiment, if one or more of the objects within the cardiac model 30 are colored to reflect the magnitudes and/or nature of various properties of the object, the colors of each object at or within a given tolerance of the model slice plane may reflected in the representation of the object within the augmented echo image

If a textual label is used to uniquely identify a particular object within the cardiac model 30, the same textual label (or an abbreviation thereof) may be used within the augmented echo image 56, 58 to identify the corresponding representation of that object. Such identification, through the use of a color and/or textual label may be equally used with both structural objects, such as volumetric regions of the model, and non-structural objects, such as lesion markers, electrodes, or other points of interest.

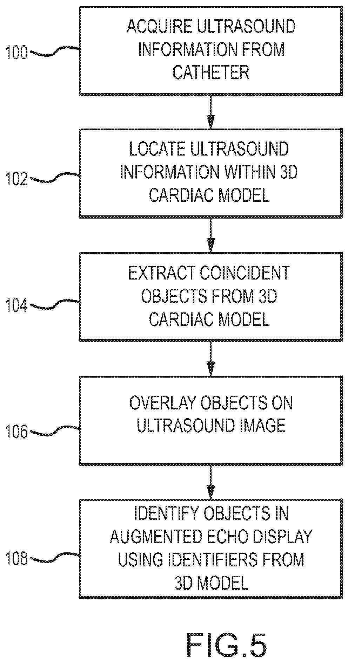

Fig. 5 generally illustrates a flow chart for an embodiment of a method of automatically identifying objects in an ultrasound image. In step 100, the system acquires ultrasound information from a catheter, such as an intracardiac echo (ICE) catheter. Such information may, for example, come from a radially scanning ultrasound transducer or from a phased array ultrasound transducer that is located near or at the distal end of a catheter in a subject's heart. The received information may be capable of being visualized in a two dimensional image, where increasingly acoustically reflective tissue may be displayed using, for example, increasingly bright monochrome colors.

In step 102, the acquired ultrasound information is located within a 3-D model of the subject's cardiac anatomy. In an embodiment, locating the ultrasound information may involve identifying the position and orientation of a representation of an ultrasound transducer within the model such that the representation reflects the position and orientation of an actual transducer within a subject's actual cardiac anatomy (as it may be perceived by a positioning system such as the Ensite NavX™ System, commercialized by St. Jude Medical, Inc.). By accurately positioning the transducer representation within the model, the system may likewise be capable of locating the 2-D ultrasound image projection within the 3-D model. In an embodiment where the model was created using a real time positioning system associated with the physical patient, or where the model has been previously registered to the catheter positioning system, no explicit registration may be required. However, if the cardiac model was created apart from the operative procedure, or was created using another imaging means (e.g., CT or MRI), the model may need to be explicitly registered to the catheter positioning system (or vice-versa).

Where an explicit registration between the cardiac model and positioning system is needed, the system may, for example, use/employ rigid body registration techniques that scale and/or rotate the model to align with the anatomy. Alternatively, registration may occur through the use of a dynamic registration tool, such as the Ensite Fusion™ Registration Tool, commercialized by St. Jude Medical, Inc. After the model is registered with the subject's cardiac anatomy (either explicitly or implicitly), the origin and orientation of the ultrasound information may be located within the cardiac anatomy using the positioning system, and located within the model using the established registration.

In an embodiment, the model may contain a plurality of structural or non-structural objects such as volumetric regions or other items of interest, where each object may be identified within the model using a separate identifier, such as a distinct color and/or textual label. In step 104, objects coincident with the plane of the ultrasound information may be extracted from the 3-D cardiac model. Such objects may include boundary information defining the walls of certain cardiac structures, or may include other non-structural objects of interest to the physician that lie within a given tolerance of the plane. In Step 106, the extracted objects may be overlaid on an independent visualization of the 2-D ultrasound information. This overlay may result in a composite image that provides more clearly identified structural boundaries or identification of features than the ultrasound alone.

Finally, in step 108, the overlaid objects in the composite image may be identified using the same or similar identifiers used for a particular corresponding object associated with the 3-D model. For example, if a particular anatomic structure was identified using a particular color, the corresponding boundary lines in the composite image would be identified using the same color. Likewise, if a particular textual label or symbol were used in the model, the same label or symbol would be used in the composite image.

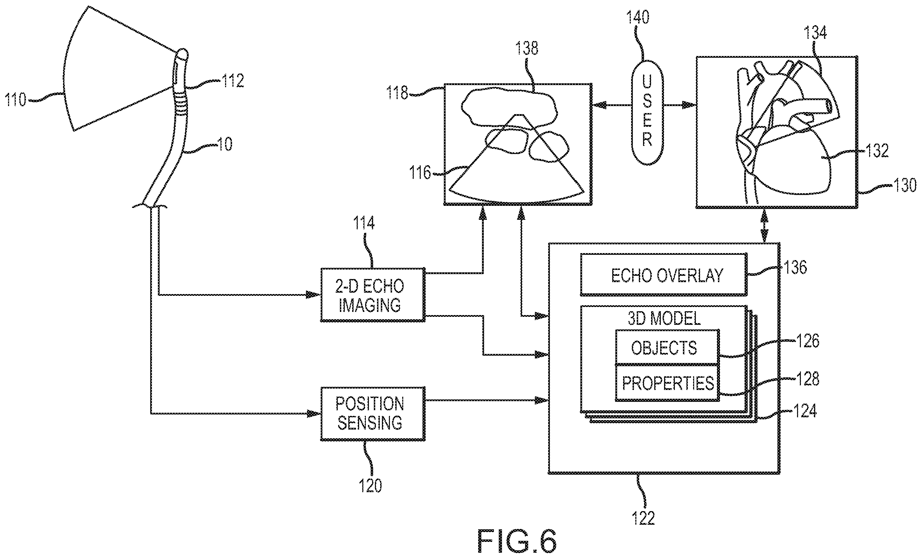

Fig. 6 illustrates a schematic of a system for identifying objects in an ultrasound image. As shown, the system may include a catheter 10, such as an ICE catheter, that is capable of projecting and receiving ultrasound information 110. The ultrasound information 110 may be transmitted/received using, for example, a phased array ultrasound transducer or a radially scanning ultrasound transducer. A distal portion of the catheter 10 may further be configured with one or more position sensors 112 configured to receive an external signal from which a position and orientation may be derived. The one or more position sensors may include, for example, electrodes configured to monitor an externally generated electric field, such as with the EnSite NavX™ System, or may include magnetically responsive coils configured to monitor an externally generated magnetic field, such as with the Medical Positioning System (gMPS).

In an embodiment, the catheter 10 may provide the ultrasound information 110 to a 2-D echo imaging system 114. The echo imaging system 114 may be configured to convert the received ultrasound information into an ultrasound image 116 that may be capable of being displayed on a monitor 118.

The catheter 10, may likewise provide a signal from each of the one or more position sensors 112 to a position sensing system 120, which may be configured to derive position and orientation of the distal portion of the catheter 10. The position sensing system 120 may be configured to provide the 3D position and orientation of the catheter 10 (with up to six degrees of freedom) to a visualization processor 122 that may maintain a 3D model 124 of a cardiac anatomy. The 3D model 124 may include a plurality of objects 126, and each object may have one or more properties 128 that define characteristics of that object 126, or characteristics of points within the object 126. In an embodiment, the visualization processor 122 may use the provided position and orientation of the distal portion of the catheter 10 to locate a representation of the catheter within the 3D model 124 of a cardiac anatomy.

In an embodiment, the echo imaging system 114 may additionally provide a representation of the ultrasound information to the visualization processor 122. Using the position and orientation of the distal portion of the catheter 10, the visualization processor 122 may locate the representation of the ultrasound information within the model 124.

The visualization processor 122 may be configured to provide a representation of the 3D model 124 to a display 130. The representation may include a display of the maintained 3D model (including anatomical structure 132), and may further include a located representation of the ultrasound information within the model (e.g., ultrasound image 134).

The visualization processor 122 may further include an echo overlay sub-processor 136. This sub-processor may allow the visualization processor 122 to extract objects within the plane of the ultrasound information (or within a specified tolerance of the plane) and generate an overlay image 138. The echo overlay sub-processor may further be configured to display the overlay image 138 with the ultrasound image 116 on monitor 118. When generating the overlay, the echo overlay sub-processor 136 may be configured to maintain existing references between each object 126 and any properties 128 associated with that object. By maintaining such references, objects within the overlay 138 may be uniquely identified (e.g., through specific colors, or textual references) in a similar manner as they are in the representation of the 3D model 124 on display 130.

As further illustrated in Fig. 6, the system allows a user 140 to interact with both the display of the 3D model 130 or the display of the augmented echo image 118 to mark features of interest to the user. In an embodiment, the user 140 may identify points within either image through the use of labels, markers, or other such tags. These objects may be useful in identifying, for example, lesion points or structural abnormalities.

In an embodiment, the user may create a non-structural object, such as a marker, by selecting the intended location for the marker in either image. Once the marker has been set within the image, the visualization processor may transfer the location coordinates from the image to the 3D model 124 as an object 126. If the catheter 10 is subsequently moved so that the plane of the ultrasound information 110 no longer contains the set object, the object may then disappear from the augmented echo display 118 (though may still be visible in the 3D model display 130). If the ultrasound plane is moved back to a position and/or orientation to contain the set point again, the object may be presented via the overlay 138 as similar to any other object within the plane.

In an embodiment, the 2-D echo imaging system 114, the position sensing system 120, and the visualization processor 122 may all be separate components that are in communication with each other through, for example, electrical or RF means. In another embodiment, any two or more of the systems or processors may reside within a single multipurpose computer. The physical relationship of the systems and/or processors should not be construed to limit this scope of the invention.

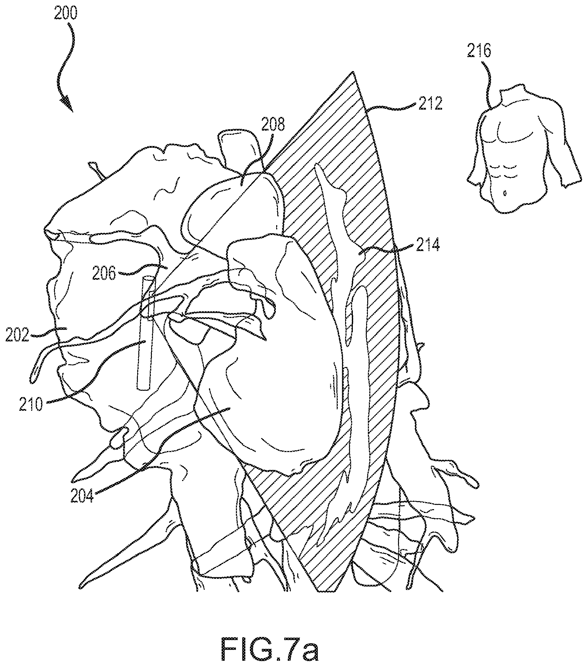

Fig. 7a illustrates a more complex segmented cardiac model 200 that may be assembled from CT or MRI slices of a subject's cardiac anatomy. As illustrated, the model 200 shows the right atrium 202, left atrium 204, aorta 206, and pulmonary artery 208. In an embodiment, various structures within the model 200 may be identified using distinct colors. For example, the right atrium 202 may be a blue color, the left atrium 204 may be a beige color, the aorta 206 may be magenta, and the pulmonary artery 208 may be red.

A representation of a catheter 210 may further be located within the model of the heart 200 and positioned and oriented according to the sensed position of the actual catheter within the subject. As illustrated, the catheter representation 210 may project a 2-D visualization of the ultrasound information 212 received by the actual catheter. If displayed, the ultrasound image 212 may likewise be oriented within the 3-D model according to a sensed position and orientation of the actual catheter within the subject. The ultrasound image 212 may contain at least one portion of lighter contrast (e.g., area 214) that may represent tissues of greater density within the subject.

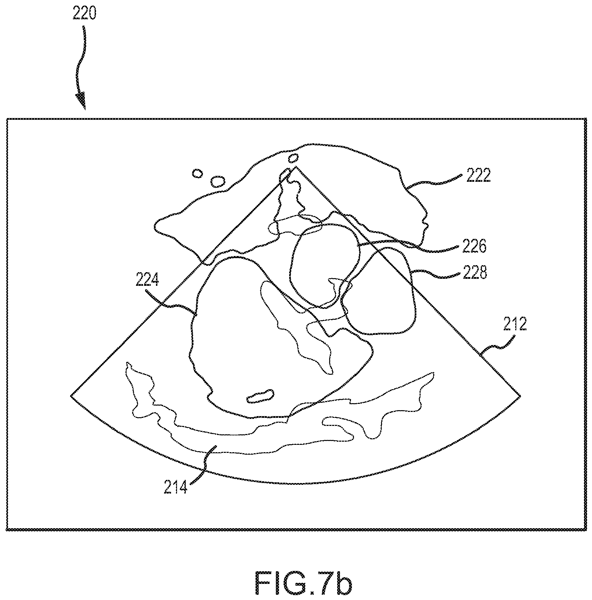

As illustrated in Fig. 7b, the ultrasound image 212 from Fig. 5a may be displayed independently from the model 200. Within the ultrasound image 212, there may likewise be at least one portion of lighter contrast (e.g., area 214) representing tissue of greater acoustic reflectivity within the subject. Additionally, boundary information, or other objects within the model 200, and coincident with the plane of the ultrasound image 212, may be extracted from the model 200 and overlaid on the ultrasound image 212 to form an augmented echo image 220. Such boundary information may include boundary lines that are identifiable as belonging to the right atrium 222, left atrium 224, aorta 226, and pulmonary artery 228.

Within the augmented echo image 220, the various other structural objects may be identified using the same or similar identifiers provided in connection with the cardiac model. For example, as shown in Fig. 5b, the boundary lines representing the right atrium 222 may be colored a blue color to match the color used for the right atrium 202 in the model 200. Similarly, the left atrium boundary lines 224 may be colored beige, the aorta boundary lines 226 may be magenta, and the pulmonary artery boundary lines 228 may be red.

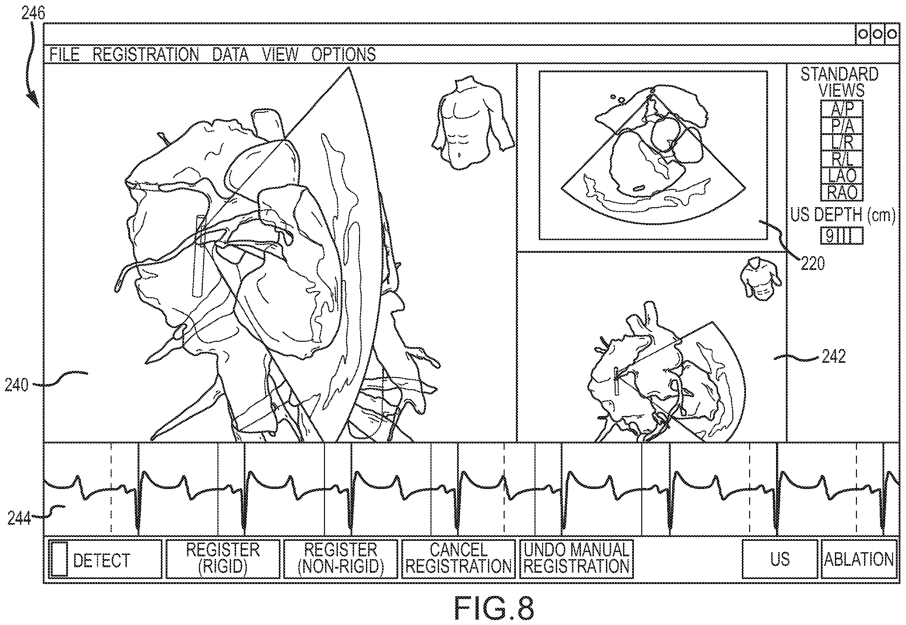

As illustrated in Fig 8, various views (e.g., views 240, 242) of the cardiac model 200, along with the augmented echo image 220 and other vital information 244, may be displayed in a single consolidated display environment 246.

While numerous embodiments of this disclosure have been described above with a certain degree of particularity, those skilled in the art could make numerous alterations to the disclosed embodiments without departing from the scope of the invention. All directional references (e.g., plus, minus, upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting.

**Claims**

1. A method of identifying objects in an ultrasound image (40, 42) comprising:
   using a processor, configured to receive ultrasound information which may be converted into an ultrasound image (116), to:

   locate (102) the ultrasound information within a three-dimensional cardiac model (30) having two or more distinct objects, where each distinct object has a unique identifier;
   extract objects coincident with the plane of the ultrasound information from the three-dimensional cardiac model (30), such objects including two-dimensional boundary information (50) that is coincident between the model and the plane containing ultrasound information, the boundary information corresponding to at least one distinct object of the model (30);
   overlaying the extracted objects on an independent visualization of the ultrasound information;
   display a two-dimensional augmented echo image (58) including the visual representation of ultrasound information and boundary lines representing the extracted, coincident boundary information; and
   identify (108) the at least one distinct object within the visual representation of the ultrasound information using an identifier corresponding to the identifier provided for that object in the three-dimensional cardiac model (30).

2. The method of claim 1, wherein using the processor to locate the ultrasound information includes:

   receiving a signal that is indicative of a position and orientation of the ultrasound information; and,
   using the signal to determine a plane containing the ultrasound information within the three-dimensional cardiac model (30).

3. The method of claim 2, wherein the signal indicative of a position and orientation is provided by a coil or electrode (18, 20, 22) responsive to a magnetic field.

4. The method of any one of claims 1 to 3, wherein an object is a volumetric region of the cardiac model (30); each of two or more volumetric regions of the cardiac model (30) is colored or provided with a unique color identifier; and wherein using the processor to identify the at least one distinct object within the representation of the ultrasound information includes coloring the boundary lines according to the color identifiers of the volumetric regions within the cardiac model (30).

5. The method of any one of claims 1 to 4, further comprising displaying the augmented echo image and a visualization of the three-dimensional cardiac model in a consolidated display.

6. The method of any one of claims 1 to 5, wherein an object is a volumetric region of the cardiac model (30), a marker identifying a physical point of interest, an electrophysiological marker, or a representation of a catheter, electrode, or magnetically responsive coil.

7. The method of any one of claims 1 to 6, wherein a unique identifier comprises at least_one of a property of the object, a color identifier, and a textual label.

8. The method of any one of claims 1 to 7, wherein at least one object within the three-dimensional cardiac model (30) is specified via the visual representation of the ultrasound information.

9. The method of any one of claims 1 to 8, wherein the object specified via the visual representation of the ultrasound information is identified within the three-dimensional cardiac model (30) using an identifier provided for that object in the visual representation of the ultrasound.

10. A system for identifying objects in an ultrasound image comprising:

    a three-dimensional cardiac model (30) having two or more distinct objects, wherein each distinct object has a unique identifier;
    a catheter (10, 38) configured to provide ultrasound information which may be converted into an ultrasound image (116);
    a display device (26); and
    a processor (24) configured to receive the ultrasound information from the catheter (10, 38), locate the ultrasound information within the three-dimensional cardiac model (30), extract objects coincident with the plane of the ultrasound information from the three-dimensional cardiac model (30), such objects including two dimensional boundary information that is coincident between the model and a plane containing ultrasound information, the boundary information corresponding to at least one distinct object of the model (30), overlay the extracted objects on an independent visualization of the ultrasound information the two dimensional boundary information, to form an augmented echo image, identify the boundary information within the augmented echo image using identifiers provided within the three dimensional cardiac model (30), and display the augmented echo image on the display device (26).

11. The system of claim 10, wherein the processor (26) is further configured to register the three dimensional cardiac model (30) to the subject's actual anatomy.

12. The system of any one of claims 10 or 11, wherein the processor (26) is further configured to provide a representation of the catheter within the three dimensional cardiac model (30) based on position information provided by an electrode or coil (18, 20, 22) associated with the catheter.

13. The system of any one of claims 10 to 12, wherein the identifiers provided within the three dimensional cardiac model (30) are one or more colors each relating to distinct anatomic structures.

14. The system of any one of claims 10 to 13, wherein the processor (26) is further configured to overlay a non-structural object from the three dimensional cardiac model (30) onto the visualization of the ultrasound information.

15. The system of any one of claims 10 to 14, wherein the display device (26) is configured to receive an indication of an object via the display of the augmented echo image; and further wherein the processor is configured to locate the indicated object within the three dimensional cardiac model (30).

**Patentansprüche**

1. Verfahren zum Identifizieren von Objekten in einem Ultraschallbild (40, 42), mit:
   Verwenden eines Prozessors, der konfiguriert ist zum Empfangen von Ultraschallinformation, die in ein Ultraschallbild (116) umgewandelt werden kann, zum:

   Lokalisieren (102) der Ultraschallinformation innerhalb eines dreidimensionalen Herzmodells (30), das zwei oder mehr individuelle Objekte aufweist, wobei jedes individuelle Objekt einen eindeutigen Identifikator hat;
   Extrahieren von Objekten, die mit der Ebene der Ultraschallinformation zusammenfallen, aus dem dreidimensionalen Herzmodell (30), wobei derartige Objekte eine zweidimensionale Grenzinformation (50) aufweisen, die zwischen dem Modell und der Ebene, die die Ultraschallinformation aufweist, übereinstimmt, wobei die Grenzinformation mindestens einem individuellen Objekt des Modells (30) entspricht;
   Überlagern der extrahieren Objekte auf eine unabhängige Visualisierung der Ultraschallinformation;
   Anzeigen eines zweidimensionalen erweiterten Echobilds (58), das die visuelle Darstellung der Ultraschallinformation und Grenzlinien, die die extrahierte übereinstimmende Grenzinformation darstellen, aufweist; und
   Identifizieren (108) des mindestens einen individuellen Objekts innerhalb der visuellen Darstellung der Ultraschallinformation unter Verwendung eines Identifikators, der dem Identifikator entspricht, der für dieses Objekt vorgesehen ist, in dem dreidimensionalen Herzmodell (30).

2. Verfahren nach Anspruch 1, bei dem das Verwenden des Prozessors zum Lokalisieren der Ultraschallinformation aufweist:

   Empfangen eines Signals, das für eine Position und Orientierung der Ultraschallinformation kennzeichnend ist; und
   Verwenden des Signals zum Bestimmen einer Ebene, die die Ultraschallinformation aufweist, innerhalb des dreidimensionalen Herzmodells (30).

3. Verfahren nach Anspruch 2, bei dem das Signal, das für eine Position und Orientierung kennzeichnend ist, von einer Spule oder Elektrode (18, 20, 22), die auf ein Magnetfeld antwortet, bereitgestellt wird.

4. Verfahren nach einem der Ansprüche 1 bis 3, bei dem ein Objekt eine Volumenregion des Herzmodells (30) ist;

wobei jede von zwei oder von mehreren Volumenregionen des Herzmodells (30) eingefärbt oder mit einem eindeutigen Farbidentifikator versehen ist; und

wobei das Verwenden des Prozessors zum Identifizieren des mindestens einen individuellen Objekts innerhalb der Darstellung der Ultraschallinformation ein Einfärben der Grenzlinien aufweist gemäß den Farbidentifikatoren der Volumenregionen innerhalb des Herzmodells (30).

5. Verfahren nach einem der Ansprüche 1 bis 4, ferner mit einem Anzeigen des erweiterten Echobilds und einer Visualisierung des dreidimensionalen Herzmodells in einer konsolidierten Anzeige.

6. Verfahren nach einem der Ansprüche 1 bis 5, bei dem ein Objekt eine Volumenregion des Herzmodells (30), eine Markierung, die einen physikalischen Punkt von Interesse angibt, eine elektrophysiologische Markierung, oder eine Darstellung eines Katheters, einer Elektrode oder einer magnetisch ansprechende Spule ist.

7. Verfahren nach einem der Ansprüche 1 bis 6, bei dem ein eindeutiger Identifikator mindestens eine Eigenschaft des Objekts, einen Farbidentifikator und/oder eine textliche Kennzeichnung aufweist.

8. Verfahren nach einem der Ansprüche 1 bis 7, bei dem mindestens ein Objekt innerhalb des dreidimensionalen Herzmodells (30) über die visuelle Darstellung der Ultraschallinformation spezifiziert ist.

9. Verfahren nach einem der Ansprüche 1 bis 8, bei dem das Objekt, das über die visuelle Darstellung der Ultraschallinformation spezifiziert ist, innerhalb des dreidimensionalen Herzmodells (30) identifiziert wird, indem ein Identifikator verwendet wird, der für dieses Objekt in der visuellen Darstellung der Ultraschallinformation vorgesehen ist.

10. System zum Identifizieren von Objekten in einem Ultraschallbild, mit:

einem dreidimensionalen Herzmodell (30), das zwei oder mehrere individuelle Objekte aufweist, wobei jedes individuelle Objekt einen eindeutigen Identifikator aufweist;

einem Katheter (10, 38), der konfiguriert ist zum Bereitstellen von Ultraschallinformation, die in ein Ultraschallbild (116) umgewandelt werden kann;

einer Anzeigevorrichtung (26); und

einem Prozessor (24), der konfiguriert ist zum Empfangen der Ultraschallinformation von dem Katheter (10, 38), Lokalisieren der Ultraschallinformation innerhalb des dreidimensionalen Herzmodells (30), Extrahieren von Objekten, die mit der Ebene der Ultraschallinformation übereinstimmen, aus dem dreidimensionalen Herzmodell (30), wobei derartige Objekte eine zweidimensionale Grenzinformation aufweisen, die zwischen dem Modell und einer Ebene, die die Ultraschallinformation aufweist, übereinstimmt, und die Grenzinformation mindestens einem individuellen Objekt des Modells (30) entspricht, Überlagern der extrahierten Objekte auf eine unabhängige Visualisierung der Ultraschallinformation der zweidimensionalen Grenzinformation, um ein erweitertes Echobild zu bilden, Identifizieren der Grenzinformation innerhalb des erweiterten Echobilds unter Verwendung der Identifikatoren, die innerhalb des dreidimensionalen Herzmodells (30) vorgesehen sind, und Anzeigen des erweiterten Echobilds auf der Anzeigevorrichtung (26).

11. System nach Anspruch 10, bei dem der Prozessor (26) ferner konfiguriert ist zum Registrieren des dreidimensionalen Herzmodells (30) mit der tatsächlichen Anatomie des Subjekts.

12. System nach einem der Ansprüche 10 oder 11, bei dem der Prozessor (26) ferner konfiguriert ist zum Bereitstellen einer Darstellung des Katheters innerhalb des dreidimensionalen Herzmodells (30) basierend auf einer Positionsinformation, die von einer Elektrode oder Spule (18, 20, 22), die dem Katheter zugeordnet ist, bereitgestellt wird.

13. System nach einem der Ansprüche 10 bis 12, bei dem die Identifikatoren, die innerhalb des dreidimensionalen Herzmodells (30) bereitgestellt sind, eine oder mehrere Farben sind, die jeweils individuelle anatomische Strukturen betreffen.

14. System nach einem der Ansprüche 10 bis 13, bei dem der Prozessor (26) ferner konfiguriert ist zum Überlagern eines nicht strukturellen Objekts von dem dreidimensionalen Herzmodell (30) auf die Visualisierung der Ultraschallinformation.

15. System nach einem der Ansprüche 10 bis 14, bei dem die Anzeigevorrichtung (26) konfiguriert ist zum Empfangen einer Angabe eines Objekts über die Anzeige des erweiterten Echobilds; und ferner der Prozessor konfiguriert ist zum Lokalisieren des angegebenen Objekts innerhalb des dreidimensionalen Herzmodells (30).

**Revendications**

1. Procédé d'identification d'objets dans une image ultrasonore (40, 42) comprenant les étapes consistant à : utiliser un processeur, configuré pour recevoir des informations ultrasonores qui peuvent être converties en une image ultrasonore (116), pour :

localiser (102) les informations ultrasonores dans un modèle cardiaque en trois dimensions (30) ayant deux objets distincts ou plus, où chaque objet distinct a un identificateur unique ;
extraire des objets coïncidant avec le plan des informations ultrasonores du modèle cardiaque en trois dimensions (30), ces objets comprenant des informations de limite en deux dimensions (50) qui coïncident entre le modèle et le plan contenant les informations ultrasonores, les informations de limite correspondant à au moins un objet distinct du modèle (30) ;
superposer les objets extraits sur une visualisation indépendante des informations ultrasonores,
afficher une image d'écho augmentée en deux dimensions (58) comprenant la représentation visuelle des informations ultrasonores et des lignes de limites représentant les informations de limite coïncidentes extraites ; et
identifier (108) ledit au moins un objet distinct dans la représentation visuelle des informations ultrasonores en utilisant un identificateur correspondant à l'identificateur fourni pour cet objet dans le modèle cardiaque en trois dimensions (30).

2. Procédé selon la revendication 1, dans lequel l'utilisation du processeur pour localiser les informations ultrasonores comprend les étapes consistant à :

recevoir un signal qui est indicatif d'une position et d'une orientation des informations ultrasonores ; et, utiliser le signal pour déterminer un plan contenant les informations ultrasonores dans le modèle cardiaque en trois dimensions (30).

3. Procédé selon la revendication 2, dans lequel le signal indicatif d'une position et d'une orientation est fourni par une bobine ou une électrode (18, 20, 22) sensible à un champ magnétique.

4. Procédé selon l'une quelconque des revendications 1 à 3, dans lequel un objet est une région volumétrique du modèle cardiaque (30) ; chacune de deux ou plusieurs régions volumétriques du modèle cardiaque (30) est colorée ou dotée d'un identificateur de couleur unique ; et
dans lequel l'utilisation du processeur pour identifier ledit au moins un objet distinct dans la représentation des informations ultrasonores comprend la coloration des lignes de délimitation selon les identificateurs de couleur des régions volumétriques dans le modèle cardiaque (30).

5. Procédé selon l'une quelconque des revendications 1 à 4, comprenant en outre l'affichage de l'image d'écho augmentée et une visualisation du modèle cardiaque en trois dimensions dans un affichage consolidé.

6. Procédé selon l'une quelconque des revendications 1 à 5, dans lequel un objet est une région volumétrique du modèle cardiaque (30), un marqueur identifiant un point d'intérêt physique, un marqueur électrophysiologique ou une représentation d'un cathéter, d'une électrode ou d'une bobine à sensibilité magnétique.

7. Procédé selon l'une quelconque des revendications 1 à 6, dans lequel un identificateur unique comprend au moins une propriété de l'objet, un identificateur de couleur et une étiquette textuelle.

8. Procédé selon l'une quelconque des revendications 1 à 7, dans lequel au moins un objet dans le modèle cardiaque en trois dimensions (30) est spécifié via la représentation visuelle des informations ultrasonores.

9. Procédé selon l'une quelconque des revendications 1 à 8, dans lequel l'objet spécifié par l'intermédiaire de la représentation visuelle des informations ultrasonores est identifié dans le modèle cardiaque en trois dimensions (30) en utilisant un identificateur fourni pour cet objet dans la représentation visuelle des ultrasons.

10. Système d'identification d'objets dans une image ultrasonore comprenant

un modèle cardiaque en trois dimensions (30) ayant deux ou plusieurs objets distincts, dans lequel chaque objet distinct a un identificateur unique ;

un cathéter (10, 38) configuré pour fournir des informations ultrasonores qui peuvent être convertie en une image ultrasonore (116) ;

un dispositif d'affichage (26) ; et

un processeur (24) configuré pour recevoir les informations ultrasonores du cathéter (10, 38), localiser les informations ultrasonores dans le modèle cardiaque en trois dimensions (30), extraire les objets coïncidant avec le plan des informations ultrasonores du modèle cardiaque en trois dimensions (30), ces objets comprenant des informations de limite en deux dimensions qui coïncident entre le modèle et un plan contenant les informations ultrasonores, les informations de limite correspondant à au moins un objet distinct du modèle (30), superposer les objets extraits sur une visualisation indépendante des informations ultrasonores, les informations de limite en deux dimensions, pour former une image d'écho augmentée, identifier les informations de limite dans l'image d'écho augmentée en utilisant des identificateurs fournis dans le modèle cardiaque en trois dimensions (30), et afficher l'image d'écho augmentée sur le dispositif d'affichage (26).

11. Système selon la revendication 10, dans lequel le processeur (26) est en outre configuré pour enregistrer le modèle cardiaque en trois dimensions (30) sur l'anatomie réelle du sujet.

12. Système selon l'une quelconque des revendications 10 ou 11, dans lequel le processeur (26) est en outre configuré pour fournir une représentation du cathéter dans le modèle cardiaque en trois dimensions (30) sur la base des informations de position fournies par une électrode ou une bobine (18, 20, 22) associée au cathéter.

13. Système selon l'une quelconque des revendications 10 à 12, dans lequel les identificateurs fournis dans le modèle cardiaque en trois dimensions (30) sont une ou plusieurs couleurs se rapportant chacune à des structures anatomiques distinctes.

14. Système selon l'une quelconque des revendications 10 à 13, dans lequel le processeur (26) est en outre configuré pour superposer un objet non structurel du modèle cardiaque en trois dimensions (30) sur la visualisation des informations ultrasonores.

15. Système selon l'une quelconque des revendications 10 à 14, dans lequel le dispositif d'affichage (26) est configuré pour recevoir une indication d'un objet via l'affichage de l'image d'écho augmentée ; et en outre dans lequel le processeur est configuré pour localiser l'objet indiqué dans le modèle cardiaque en trois dimensions (30).

FIG.1

FIG.2a

FIG.2b

FIG.3a

FIG.3b

FIG.4a

FIG.4b

FIG.5

FIG.6

FIG.7a

FIG.7b

FIG.8

EP 2 632 341 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005203394 A1
- EP 1504713 A1
- US 2006122514 A1
- US 7263397 B
- US 7386339 B